(19) Europäisches Patentamt
European Patent Office
Office européen des brevets

(11) EP 1 844 817 A2

(12) DEMANDE DE BREVET EUROPEEN

(43) Date de publication:
**17.10.2007 Bulletin 2007/42**

(21) Numéro de dépôt: **07105829.1**

(22) Date de dépôt: **06.04.2007**

(51) Int Cl.:
***A61Q 5/12*** (2006.01) ***A61K 8/37*** (2006.01)
***A61K 8/06*** (2006.01) ***A61K 8/36*** (2006.01)
***A61K 8/31*** (2006.01) ***A61K 8/41*** (2006.01)
***A61K 8/58*** (2006.01) ***A61K 8/894*** (2006.01)
***A61K 8/97*** (2006.01)

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK YU**

(30) Priorité: **13.04.2006 FR 0603319**

(71) Demandeur: **L'ORÉAL**
**75008 Paris (FR)**

(72) Inventeurs:
- **Samain, Henri**
**91570 Bievres (FR)**
- **Hercouet, Leïla**
**93360 Neuilly Plaisance (FR)**
- **Fack, Géraldine**
**92300 Levallois (FR)**

(74) Mandataire: **Fevrier, Murielle Françoise E.**
**L'OREAL - D.I.P.I. - River Plaza**
**25-29 Quai Aulagnier**
**92665 Asnières-sur-Seine (FR)**

(54) **Emulsion eau-dans-l'huile pour le traitement des fibres kératiniques comprenant un monomère cyanoacrylate et de l'ammoniaque**

(57) La présente invention concerne une émulsion eau dans huile qui comprend une phase aqueuse comprenant au moins un tensio-actif ; au moins un agent alcalin azoté de type ammoniaque ou amine et une phase huileuse comprenant un ou plusieurs monomères cyanoacrylates

L'émulsion eau dans l'huile de la présente invention permet de réduire les odeurs d'ammoniaque à l'utilisation.

EP 1 844 817 A2

## Description

La présente invention a pour objet une émulsion eau dans l'huile pour le traitement des fibres kératiniques, en particulier des fibres kératiniques telles que les cheveux, comprenant au moins un monomère cyanoacrylate.

Il est connu de colorer les fibres kératiniques à partir de précurseurs de colorants pour obtenir des colorations permanentes. Ce type de coloration nécessite l'utilisation d'ammoniaque afin de favoriser la pénétration des précurseurs de colorants dans la fibre kératinique et la réaction oxydative responsable de l'apparition de la couleur à l'intérieur de cette fibre.

L'utilisation de ces compositions à base d'ammoniaque présentent un désagrément du à l'odeur à la présence d'ammoniaque. L'odeur de ces compositions est d'autant plus forte que le pH de la composition est élevé. Le même désagrément est présent pour d'autres types de traitements capillaires tels que la permanente, le défrisage, la décoloration ainsi que pour d'autres compositions dans lesquelles l'ammoniaque a été remplacée par d'autres agents azotés alcalins tel que l'éthanolamine, etc.

Ainsi, le but de la présente invention est d'obtenir des compositions à base d'ammoniaque ou de tout autre composé azoté alcalin tout aussi efficace que celles existantes mais sans les désagréments liés à l'odeur lors de l'utilisation.

Ce but est atteint avec la présente invention qui a pour objet une émulsion eau dans huile qui comprend une phase aqueuse comprenant au moins un tensio-actif ; au moins un agent alcalin azoté de type ammoniaque ou amine et une phase huileuse comprenant un ou plusieurs monomères cyanoacrylates capables de polymériser par voie anionique en présence d'un agent nucléophile.

L'émulsion eau dans l'huile de la présente invention permet de réduire de façon considérable l'odeur typique de l'ammoniaque lors de l'utilisation sur les fibres kératiniques.

La présente invention a aussi pour objet un procédé de traitement des fibres kératiniques qui comprend l'application sur les fibres kératiniques de l'émulsion de l'invention ainsi que l'utilisation de cette émulsion pour le traitement des fibres kératiniques tels que les cheveux.

L'émulsion de l'invention contient une phase huileuse. Cette phase huileuse peut comprendre un ou plusieurs solvants organiques. Les solvants organiques sont généralement choisis parmi les composés liquides à la température de 25°C et sous 105 Pa (760mm de Hg).

Dans le cadre de l'invention le monomère cyanoacrylate et le solvant organique sont distincts. La phase huileuse peut être constitué par le monomère électrophile.

Le ou les solvants organiques sont par exemple choisis parmi :

les alcools aromatiques tels que l'alcool benzylique ; les alcools gras liquides , notamment en C10-C30; les polyols modifiés ou non tels que le glycérol, le glycol, le propylène glycol, le dipropylène glycol, le butylène glycol, le butyle diglycol ; les silicones volatiles telles que la cylopentasiloxane, la cyclohexasiloxane, les polydiméthylsiloxanes modifiées ou non par des fonctions alkyle et/ou amine et/ou imine et/ou fluoroalkyl et/ou carboxylique et/ou betaïne et/ou ammonium quaternaire, les polydiméthylsiloxanes modifiées liquides, les huiles minérales, organiques ou végétales, les alcanes et plus particulièrement les alcanes de C5 à C10 ; les acides gras liquides, les esters gras liquides et plus particulièrement les benzoates ou les salicylates d'alcool gras liquides.

Le solvant organique est de préférence choisi parmi les huiles organiques ; les silicones telles que les silicones volatiles, les gommes ou huiles de silicones aminés ou non et leurs mélanges ; les huiles minérales ; les huiles végétales telles que les huiles d'olive, de ricin, de colza, de coprah, de germe de blé, d'amande douce, d'avocat, de macadamia, d'abricot, de carthame, de noix de bancoulier, de camélina, de tamanu, de citron ou encore des composés organiques tels que des alcanes en C5-C10, l'acétone, la méthyléthylcétone, les esters d'acides en C1-C20 liquides et d'alcools en C1-C8 tels que l'acétate de méthyle, l'acétate de butyle, l'acétate d'éthyle et le myristate d'isopropyle, le diméthoxyéthane, le diéthoxyéthane, les alcools gras liquides en C10-C30 tels que l'alcool oléique, les esters d'alcools gras en C10-C30 liquides tels que les benzoates d'alcool gras en C10-C30 et leurs mélanges ; l'huile de polybutène, l'isononanoate d'isononyle, le malate d'isostéaryle, le tétra-isostéarate de pentaérythrityle, le trimélate de tridécyle, le mélange cyclopentasiloxane (14,7% en poids)/polydiméthylsiloxane dihydroxylé en positions $\alpha$ et $\omega$ (85,3% en poids), ou leurs mélanges.

Selon un mode de réalisation préféré, le solvant organique est constitué par une silicone ou un mélange de silicones tels que les polydiméthylsiloxanes liquides et les polydiméthylsiloxanes modifiées liquides, leur viscosité à 25°c est comprise entre 0.1 cst et 1 000 000cst et plus préférentiellement entre 1 cst et 30 000cst.

On citera de préférence les huiles et les mélanges d'huiles suivantes :

- le mélange de polydiméthylsiloxane alpha-omega-dihydroxylé/cyclopentadiméthylsiloxane (14,7/85,3) commercialisé par Dow Corning sous le nom de DC 1501 Fluid

- le mélange de polydiméthylsiloxane alpha-omega-dihydroxylé/polydiméthylsiloxane commercialisé par Dow Corning sous le nom de DC 1503 Fluid
- le mélange de diméthicone /cyclopentadiméthylsiloxane commercialisé par Dow Corning sous le nom de DC 1411 Fluid ou celui commercialisé par Bayer sous le nom SF1214 ;
- la cyclopentadiméthylsiloxane commercialisée par Dow Corning sous le nom de DC245 Fluid ; et les mélanges respectifs de ces huiles.

Selon un mode de réalisation de l'invention, la phase huileuse comprend préférentiellement une silicone volatile, seule ou en mélange avec une autre silicone.
Les silicones volatiles utilisables dans l'invention sont des silicones linéaires ou cycliques, ayant une viscosité à température ambiante et sous pression atmosphérique, inférieure à 8 mm2/s (8 cSt).
La viscosité est mesurée de préférence par viscosimétrie capillaire, par exemple, à l'aide d'un viscosimètre capillaire, notamment de type Ubbelohde, à une température de 25 °C, selon la norme ASTM D445-97. On peut aussi utiliser la méthode dite de la chute de la bille.
Les silicones volatiles présentent généralement un point d'ébullition compris entre 60 °C et 260° C, et sont plus particulièrement choisies parmi :

(i) les silicones volatiles cycliques comportant de 3 à 7 atomes de silicium et, de préférence, 4 à 5. Il s'agit, par exemple, de l'octaméthylcyclotétrasiloxane commercialisé notamment sous le nom de "VOLATILE SILICONE 7207" par UNION CARBIDE ou "SILBIONE® 70045 V 2" par RHODIA, le décaméthylcyclopentasiloxane communément appelée D5, commercialisé sous le nom de "VOLATILE SILICONE 7158" par UNION CARBIDE, "SILBIONE® 70045 V 5" par RHODIA ou sous le nom DC245 Fluid par DOW CORNING, ainsi que leurs mélanges.
On peut également citer les cyclocopolymères du type diméthylsiloxane/méthylalkylsiloxane, tel que la "SILICONE VOLATILE FZ 3109" commercialisée par la société UNION CARBIDE, de structure chimique :

avec D :

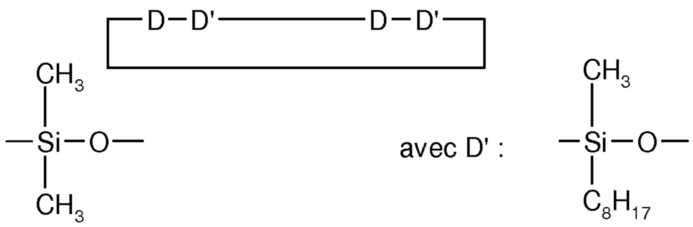

avec D' :

On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5 mm2/s à 25 °C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, p. 27-32 - TODD & BYERS "Volatile Silicone fluids for cosmetics".

La phase huileuse est généralement comprise dans l'émulsion à raison de 0,1 et 50 % en poids de l'émulsion ; plus préférentiellement entre 1% et 30% en poids d'émulsion, tout particulièrement entre 5 et 25 %.
Lorsque la phase huileuse comprend une ou plusieurs silicones volatiles, ces silicones sont généralement comprises entre 0,1 et 30 % en poids, de préférence comprise entre 5 et 20 % en poids, et préférentiellement entre 8 et 15 % en poids par rapport au poids total d'émulsion.
Le tensio-actif utile dans l'émulsion de l'invention peut être n'importe quel tensio-actif connu de la technique pour former des émulsions eau dans l'huile. Ce tensio-actif peut être anionique, amphothère, cationique ou non ionique.
A titre de tensio-actifs utiles dans l'invention, on peut citer les tensioactifs anioniques non siliconés notamment les sels des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, $\alpha$ -oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Les sels de

ces composés sont par exemple des sels alcalins, notamment de sodium, sels d'ammonium, sels d'amines, sels d'aminoalcools ou sels de magnésium. Parmi les tensioactifs anioniques on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides éthers carboxyliques polyoxyalkylénés et leurs sels, en particulier ceux comportant de 2 à 50 groupements oxyde d'éthylène, et leurs mélanges. Les tensioactifs anioniques du type acides ou sels d'éthers carboxyliques polyoxyalkylénés sont en particulier ceux qui répondent à la formule (1) suivante :

$$R_1-(OC_2H_4)_n-OCH_2COOA \qquad (1)$$

dans laquelle :

R1 représente un groupement alkyle ou alkylaryle, et n est un nombre entier ou décimal (valeur moyenne) pouvant varier de 2 à 24 et de préférence de 3 à 10, le radical alkyle ayant entre 6 et 20 atomes de carbone environ, et aryle désignant de préférence phényle,

A représente un atome d'hydrogène, un ammonium, ion sodium, potassium, lithium ou magnésium ou un reste monoéthanolamine ou triéthanolamine. On peut également utiliser des mélanges de composés de formule (1) en particulier des mélanges dans lesquels les groupements R1 sont différents.

Parmi tous ces tensioactifs anioniques, on préfère utiliser les sels d'alkylsulfates et d'alkyléthersulfates, ainsi que leurs mélanges.

A titre de tensio-actifs amphotères non siliconés, on peut citer des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant par exemple carboxylate, sulfonate, sulfate, phosphate ou phosphonate ; on peut citer encore les alkyl (C8-C20) bétaïnes, les sulfobétaïnes, les alkyl (C8-C20) amidoalkyl (C1-C6) bétaïnes ou les alkyl (C8-C20) amidoalkyl (C1-C6) sulfobétaïnes.

Parmi les dérivés d'amines, on peut citer les produits commercialisés sous les dénomination MIRANOL, tels que décrits dans les brevets US-2 528 378 et US-2 781 354 et de structures :

R2 -CONHCH2CH2 -N(R3)(R4)(CH2COO-) (2)

dans laquelle : R2 désigne un radical alkyle dérivé d'un acide R2-COOH présent dans l'huile de coprah hydrolysée, un radical heptyle, nonyle ou undécyle, R3 désigne un groupement bêta-hydroxyéthyle et R4 un groupement carboxyméthyle ; et

R5-CONHCH2CH2-N(B)(C) (3)

dans laquelle

B représente -CH2CH2OX', C représente -(CH2)z -Y', avec z = 1 ou 2,
X' désigne le groupement -CH2CH2-COOH ou un atome d'hydrogène
Y' désigne -COOH ou le radical -CH2 - CHOH - S03H
R5 désigne un radical alkyle d'un acide R9 -COOH présent dans l'huile de coprah ou dans l'huile de lin hydrolysée, un radical alkyle, notamment en C7, C9, C11 ou C13, un radical alkyle en C17 et sa forme iso, un radical C17 insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.

A titre d'exemple on peut citer le cocoamphodiacetate commercialisé sous la dénomination commerciale MIRANOL C2M concentré par la société RHONE POULENC.

A titre de tensioactif(s) non ionique(s) non siliconé(s), on peut citer les tensioactifs non-ioniques bien connus en soi (voir notamment à cet égard "Handbook of Surfactants" par M.R. PORTER, éditions Blackie & Son (Glasgow and London), 1991, pp 116-178). Ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant

par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C10 - C14) amines ou les oxydes de N-acylaminopropylmorpholine. Selon un mode de réalisation de l'invention, les alkylpolyglycosides constituent des tensio-actifs non-ioniques utiles dans le cadre de la présente invention.

A titre de tensioactifs cationiques non siliconé, on peut utiliser les tensioactifs bien connus en soi, tels que les sels d'amines grasses primaires, secondaires ou tertiaires, éventuellement polyoxyalkylénées, les sels d'ammonium quaternaire, et leurs mélanges.

A titre de sels d'ammonium quaternaires, on peut notamment citer,

- ceux qui présentent la formule suivante :

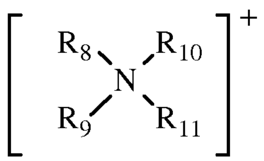 $X^-$ (VI)

dans laquelle les radicaux R8 à R11, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre et les halogènes. Les radicaux aliphatiques sont par exemple choisis parmi les radicaux alkyle, alcoxy, polyoxyalkylène (C2-C6), alkylamide, alkyl(C12-C22)amidoalkyle(C2-C6), alkyl(C12-C22)acétate, hydroxyalkyle, comportant environ de 1 à 30 atomes de carbone ; X est un anion choisi dans le groupe des halogénures, phosphates, acétates, lactates, alkyl(C2-C6)sulfates, alkyl- ou alkylaryl-sulfonates ;

- les sels d'ammonium quaternaire de l'imidazoline, comme par exemple ceux de formule suivante :

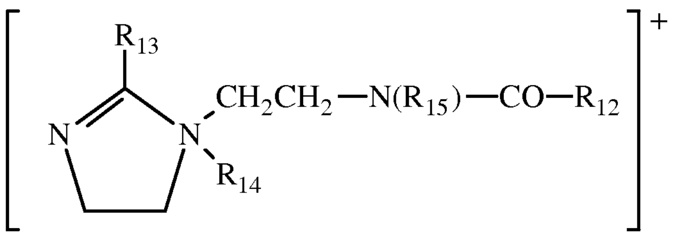 $X^-$ (VII)

dans laquelle R12 représente un radical alcényle ou alkyle comportant de 8 à 30 atomes de carbone, par exemple dérivés des acides gras du suif, R13 représente un atome d'hydrogène, un radical alkyle en C1-C4 ou un radical alcényle ou alkyle comportant de 8 à 30 atomes de carbone, R14 représente un radical alkyle en C1-C4, R15 représente un atome d'hydrogène, un radical alkyle en C1-C4, X- est un anion choisi dans le groupe des halogénures, phosphates, acétates, lactates, alkylsulfates, alkyl- ou alkylaryl-sulfonates. De préférence, R12 et R13 désignent un mélange de radicaux alcényle ou alkyle comportant de 12 à 21 atomes de carbone, par exemple dérivés des acides gras du suif, R14 désigne un radical méthyle, R15 désigne un atome d'hydrogène. Un tel produit est par exemple commercialisé sous la dénomination REWOQUAT® W 75 par la société REWO ;

- les sels de diammonium quaternaire de formule (VIII) :

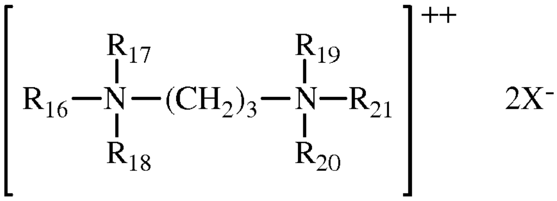

(VIII)

dans laquelle R16 désigne un radical aliphatique comportant environ de 16 à 30 atomes de carbone, R17, R18, R19, R20 et R21 , identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium ;

- les sels d'ammonium quaternaire contenant au moins une fonction ester, tels que ceux de formule (IX) suivante :

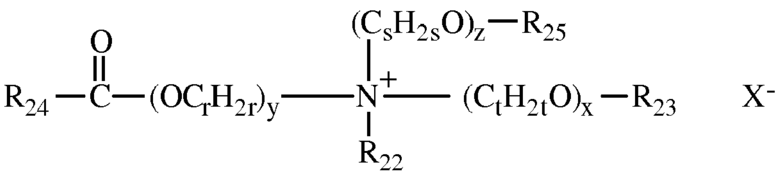

(IX)

dans laquelle :

R22 est choisi parmi les radicaux alkyles en C1-C6 et les radicaux hydroxyalkyles ou dihydroxyalkyles en C1-C6 ;
R23 est choisi parmi :

- le radical

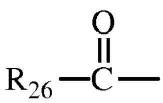

- les radicaux R27 hydrocarbonés en C1-C22, linéaires ou ramifiés, saturés ou insaturés,
- l'atome d'hydrogène,

R25 est choisi parmi :

- le radical

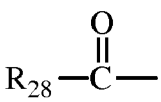

- les radicaux R29 hydrocarbonés en C1-C6, linéaires ou ramifiés, saturés ou i nsatu rés,
- l'atome d'hydrogène,

R24, R26 et R28, identiques ou différents, sont choisis parmi les radicaux hydrocarbonés en C7-C21, linéaires ou ramifiés, saturés ou insaturés ;
r, s et t, identiques ou différents, sont des entiers valant de 2 à 6 ;
y est un entier valant de 1 à 10 ;
x et z, identiques ou différents, sont des entiers valant de 0 à 10 ;

X- est un anion simple ou complexe, organique ou inorganique ;

sous réserve que la somme x + y + z vaut de 1 à 15, que lorsque x vaut 0 alors R23 désigne R27 et que lorsque z vaut 0 alors R25 désigne R29.

Les radicaux alkyles R22 peuvent être linéaires ou ramifiés et plus particulièrement linéaires.

De préférence R22 désigne un radical méthyle, éthyle, hydroxyéthyle ou dihydroxypropyle, et plus particulièrement un radical méthyle ou éthyle.

Avantageusement, la somme x + y + z vaut de 1 à 10.

Lorsque R23 est un radical R27 hydrocarboné, il peut être long et avoir de 12 à 22 atomes de carbone, ou court et avoir de 1 à 3 atomes de carbone.

Lorsque R25 est un radical R29 hydrocarboné, il a de préférence 1 à 3 atomes de carbone.

Avantageusement, R24, R26 et R28, identiques ou différents, sont choisis parmi les radicaux hydrocarbonés en C11-C21, linéaires ou ramifiés, saturés ou insaturés, et plus particulièrement parmi les radicaux alkyle et alcényle en C11-C21, linéaires ou ramifiés, saturés ou insaturés.
De préférence, x et z, identiques ou différents, valent 0 ou 1.

Avantageusement, y est égal à 1.

De préférence, r, s et t, identiques ou différents, valent 2 ou 3, et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X- est encore plus particulièrement le chlorure ou le méthylsulfate. On utilise plus particulièrement dans la composition selon l'invention, les sels d'ammonium de formule (IX) dans laquelle :

- R22 désigne un radical méthyle ou éthyle,
- x et y sont égaux à 1;
- z est égal à 0 ou 1 ;
- r, s et t sont égaux à 2 ;
- R23 est choisi parmi :
- le radical

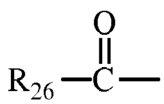

- les radicaux méthyle, éthyle ou hydrocarbonés en C14-C22,
- l'atome d'hydrogène ;
- R25 est choisi parmi :

  - le radical

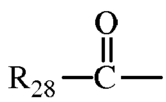

- l'atome d'hydrogène ;
- R24, R26 et R28, identiques ou différents, sont choisis parmi les radicaux hydrocarbonés en C13-C17, linéaires ou ramifiés, saturés ou insaturés, et de préférence parmi les radicaux alkyles et alcényles en C13-C17, linéaires ou ramifiés, saturés ou insaturés.

Avantageusement, les radicaux hydrocarbonés sont linéaires. On peut citer par exemple les composés de formule (IX) tels que les sels (chlorure ou méthylsulfate notamment) de diacyloxyéthyl-diméthylammonium, de diacyloxyéthylhydroxyéthylméthylammonium, de monoacyloxyéthyldihydroxyéthylméthylammonium, de triacyloxyéthylméthylammonium, de monoacyloxyéthylhydroxyéthyldiméthylammonium et leurs mélanges. Les radicaux acyles ont de préférence 14 à 18 atomes de carbone et proviennent plus particulièrement d'une huile végétale comme l'huile de palme

ou de tournesol. Lorsque le composé contient plusieurs radicaux acyles, ces derniers peuvent être identiques ou différents.

Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthanesulfonate de méthyle, le paratoluènesulfonate de méthyle, la chlorhydrine du glycol ou du glycérol.

De tels composés sont par exemple commercialisés sous les dénominations DEHYQUART® par la société HENKEL, STEPANQUAT® par la société STEPAN, NOXAMIUM® par la société CECA, REWOQUAT® WE 18 par la société REWO-WITCO.

A titre de tensioactifs cationique, on préfère le mélange de sels de mono-, di- et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyldihydroxyéthyl-méthylammonium, 45 à 60% de méthylsulfate de diacyloxyéthylhydroxyéthyl-méthylammonium et 15 à 30% de méthylsulfate de triacyloxyéthylméthylammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

Parmi les sels d'ammonium quaternaire de formule (VI), on préfère d'une part, les chlorures de tétraalkylammonium comme, par exemple, les chlorures de dialkyldiméthylammonium ou d'alkyltriméthylammonium dans lesquels le radical alkyle comporte environ de 12 à 22 atomes de carbone, en particulier les chlorures de béhényltriméthylammonium, de distéaryldiméthylammonium, de cétyltriméthylammonium, de benzyldiméthylstéarylammonium ou encore, d'autre part, le chlorure de palmitylamidopropyltriméthylammonium ou le chlorure de stéaramidopropyldiméthyl-(myristyl acétate)-ammonium commercialisé sous la dénomination CERAPHYL® 70 par la société VAN DYK.

Les tensioactifs cationiques particulièrement préférés sont choisis parmi les sels d'ammonium quaternaire, et en particulier parmi le chlorure de béhényltriméthylammonium et le chlorure de palmitylamidopropyltriméthylammonium.

Les tensio-actifs non siliconés sont généralement présents dans l'émulsion de la présente invention dans des proportions variant de 0,1% à 30% en poids de matière active ; plus préférentiellement entre 0,5% et 15% en poids de composition.

- A titre de tensioactifs utilisables dans la présente invention on peut aussi particulièrement citer les tensioactifs siliconés décrits notamment dans le brevet FR2818902. Les tensioactifs siliconés utilisables dans la présente invention sont ceux bien connus de l'homme du métier. Ils peuvent être hydrosolubles, spontanément hydrodispersibles ou non hydrosolubles. De préférence, ils sont hydrosolubles ou spontanément hydrodispersibles.
  Les tensioactifs siliconés sont, par exemple, choisis parmi les composés de formules générales suivantes :

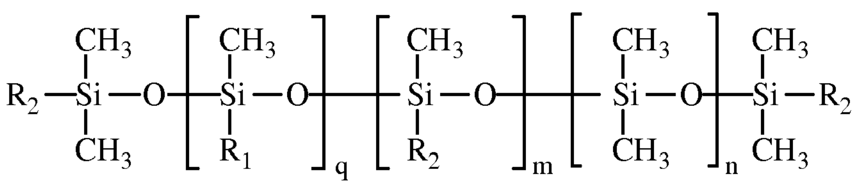

(I)

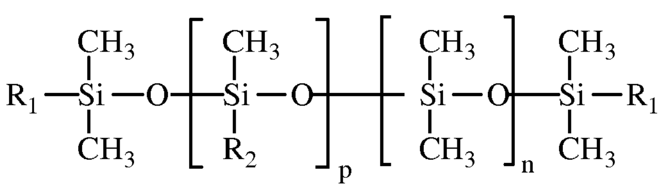

(II)

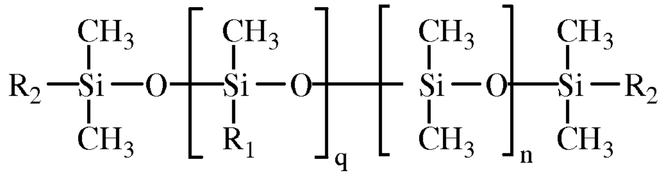

(III)

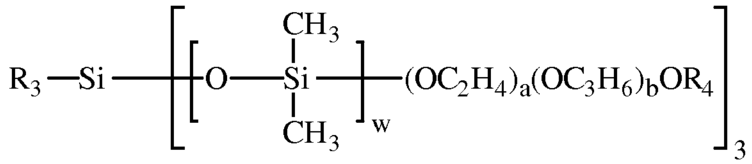

(IV)

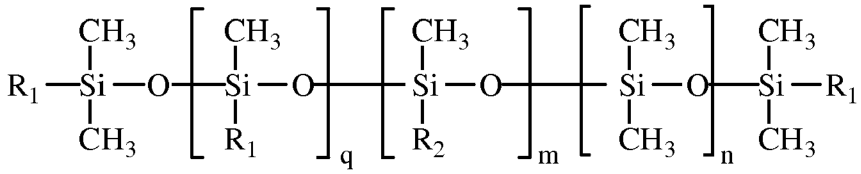

(V)

formules dans lesquelles :

- R1, identique ou différent, représente un groupe alkyle, linéaire ou ramifié, en C1-C30, ou phényle;
- R2, identique ou différent, représente -CcH2c-O-(C2H4O)a-(C3H6O)b-R5 ou -CcH2c-O-(C4H8O)a-R5;
- R3 et R4, identiques ou différents, désignent chacun un groupe alkyle, linéaire ou ramifié, en C1-C12, et de préférence un groupe méthyle ;
- R5, identique ou différent, est choisi parmi un atome d'hydrogène, un groupe alkyle, linéaire ou ramifié, comportant de 1 à 12 atomes de carbone, un groupe alcoxy, linéaire ou ramifié, comportant de 1 à 6 atomes de carbone, un groupe acyle, linéaire ou ramifié, comportant de 2 à 12 atomes de carbone, un groupe hydroxyle, -SO3M, - OCOR6, aminoalcoxy en C1-C6 éventuellement substitué sur l'amine, aminoacyle en C2-C6 éventuellement substitué sur l'amine, - NHCH2CH2COOM, -N(CH2CH2COOM)2, aminoalkyle en C1-C12 éventuellement substitué sur l'amine et sur la chaîne alkyle, carboxyacyle en C1-C30, un groupement phosphono éventuellement substitué par un ou deux groupes aminoalkyle en C1-C12 substitués, -CO(CH2)dCOOM, -OCOCHR7(CH2)dCOOM, -NHCO(CH2)dOH, -NH3Y
- M, identique ou différent, désigne un atome d'hydrogène, Na, K, Li, NH4 ou une amine organique ;
- R6 désigne un groupe alkyle, linéaire ou ramifié, en C1-C30,
- R7 désigne un atome d'hydrogène ou un groupe SO3M ;
- d varie de 1 à 10;
- m varie de 0 à 20 ;
- n varie de 0 à 500 ;
- p varie de 1 à 50 ;
- q varie de 0 à 20 ;
- a varie de 0 à 50 ;
- b varie de 0 à 50 ;
- a + b est supérieur ou égal à 1 ;
- c varie de 0 à 4 ;
- w varie de 1 à 100;
- Y représente un anion minéral ou organique monovalent tel qu'un halogénure (chlorure, bromure), un sulfate, ou un carboxylate (acétate, lactate, citrate).

De préférence, on utilise des tensioactifs siliconés répondant aux formules générales (IV) ou (VII) telles que définies ci-dessus, et plus particulièrement, ceux répondant aux formules (IV) ou (VII) dans lesquelles au moins l'une des, et de préférence toutes les conditions suivantes sont satisfaites :

- c est égal à 2 ou 3 ;
- R1 désigne le groupe méthyle ;
- R5 représente un atome d'hydrogène, un groupe méthyle ou un groupe acétyle et de préférence un atome d'hydrogène ;
- a varie de 1 à 25 et plus particulièrement de 2 à 25 ;
- b varie de 0 à 25, de préférence de 10 à 20 ;
- n varie de 0 à 100;
- p varie de 1 à 20.

Les tensioactifs siliconés les plus particulièrement préférés sont, par exemple, ceux vendus sous les dénominations commerciales FLUID DC 193 et DC 5225C par la société DOW CORNING, SILWET® L 77 par la société OSI et MAZIL® 756 par la société MAZER PPG, le mélange LAURYL PEG/PPG-18/18 METHICONE (and) POLOXAMER 407 (and) DODECENE commercialisé par Dow Corning sous le nom de DC 5200
Bien que tout type de ionicité puisse être utilisé pour les émulsions de la présente invention, les tensio-actifs présentant une HLB (hydrophilic Lipophilic Balance) inférieure à 10 sont préférés. En particulier, les tensioactifs non ioniques utilisés dans les compositions de l'invention présentent de préférence une HLB allant de 1,5 à 10, et mieux encore de 1,5 à 7. La HLB ou balance hydrophile-lipophile du ou des tensioactifs non ioniques utilisés selon l'invention est la HLB selon GRIFFIN définie dans la publication J. Soc. Cosm. Chem. 1954 (Volume 5), pages 249-256.
Le ou les tensioactifs sont contenus généralement en une quantité allant de 0,01 à 30 % en poids, de préférence de 0,1 à 30% en poids, et mieux encore de 0,2 à 15 % en poids par rapport au poids total de l'émulsion
Selon un mode de réalisation préféré, le ou les tensio-actifs présents dans l'émulsion sont choisis parmi les tensio-actifs siliconés.
Les tensio-actifs siliconés sont généralement présents dans l'émulsion de la présente invention dans des proportions variant de 0,1% à 30% en poids d'émulsion; préférentiellement entre 0,2% et 15% en poids d'émulsion.
Le ou les monomères cyanoacrylate présents dans la composition de l'invention sont de préférence choisis parmi les monomères de formule (IX) :

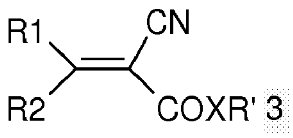

dans laquelle :

- X désigne NH, S ou O,
- R1 et R2 désignent chacun, indépendamment l'un de l'autre, un groupe peu ou non électro-attracteur (peu ou non inductif-attracteur) tel que :

  - un atome d'hydrogène,
  - un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR, - COOR, -COR, -SH, -SR, -OH, et les atomes d'halogène,
  un résidu polyorganosiloxane modifié ou non,
  - un groupement polyoxyalkylène,

R désigne un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR', -COOR', -COR', -SH, -SR', -OH, les atomes d'halogène, ou un résidu de polymère pouvant être obtenu par polymérisation radicalaire, par polycondensation ou par ouverture de cycle, R' désignant un groupe alkyle en $C_1$-$C_{10}$.
Par groupement électro-attracteur ou inductif-attracteur (-I), on entend tout groupement plus électronégatif que le carbone. On pourra se reporter à l'ouvrage PR Wells Prog. Phys. Org. Chem., Vol 6,111 (1968).

Par groupement peu ou non électro-attracteur, on entend tout groupement dont l'électronégativité est inférieure ou égale à celle du carbone.

Les groupements alcényle ou alcynyle ont de préférence 2 à 20 atomes de carbone, mieux encore de 2 à 10 atomes de carbone.

Comme groupe hydrocarboné saturé ou non, linéaire, ramifié ou cyclique, comportant de préférence de 1 à 20 atomes de carbone, on peut notamment citer les groupes alkyle, alcényle ou alcynyle linéaires ou ramifiés, tels que méthyle, éthyle, n-butyle, tert-butyle, iso-butyle, pentyle, hexyle, octyle, butényle ou butynyle ; les groupes cycloalkyle ou aromatiques.

Comme groupe hydrocarboné substitué, on peut citer par exemple les groupes hydroxyalkyle ou polyhalogénoalkyle.

A titre d'exemples de polyorganosiloxane non modifié, on peut notamment citer les polyalkylsiloxanes tels que les polydiméthylsiloxanes, les polyarylsiloxanes tels que les polyphénylsiloxanes, les polyarylalkylsiloxanes tels que les polyméthylphénylsiloxanes.

Parmi les polyorganosiloxanes modifiés, on peut notamment citer les polydiméthylsiloxanes à groupements polyoxyalkylène et / ou siloxy et/ou silanol et / ou amine et / ou imine et / ou fluoroalkyle.

Parmi les groupements polyoxyalkylène, on peut notamment citer les groupements polyoxyéthylène et les groupements polyoxypropylène ayant de préférence 1 à 200 motifs oxyalkylénés.

Parmi les groupements mono- ou polyfluoroalkyle, on peut notamment citer des groupements tels que $-(CH_2)_n-(CF_2)_m-CF_3$ ou $-(CH_2)_n-(CF_2)_m-CHF_2$ avec n = 1 à 20 et m = 1 à 20.

Les substituants R1 et R2 peuvent éventuellement être substitués par un groupement ayant une activité cosmétique. Les activités cosmétiques particulièrement utilisées sont obtenues à partir de groupements à fonctions colorantes, antioxydantes, filtres UV et conditionnantes.

A titre d'exemples de groupement à fonction colorante, on peut notamment citer les groupements azoiques, quinoniques, méthiniques, cyanométhiniques et triarylméthane.

A titre d'exemples de groupement à fonction antioxydante, on peut notamment citer les groupements de type butylhydroxyanisole (BHA), butylhydroxytoluène (BHT) ou vitamine E.

A titre d'exemples de groupement à fonction filtre UV, on peut notamment citer les groupements de types benzophénones, cinnamates, benzoates, benzylidène-camphres et dibenzoylméthanes.

A titre d'exemples de groupement à fonction conditionnante, on peut notamment citer les groupements cationiques et de type esters gras.

De préférence R1 et R2 représentent un atome d'hydrogène,

R'3 représente un atome d'hydrogène ou un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR', -COOR', -COR', -SH, -SR', -OH, les atomes d'halogène, ou un résidu de polymère pouvant être obtenu par polymérisation radicalaire, par polycondensation ou par ouverture de cycle, R' désignant un groupe alkyle en $C_1$-$C_{10}$.

De préférence R'3 est un groupe hydrocarboné saturé comportant de 1 à 10 atomes de carbone.

De préférence, X désigne O.

A titre de composés de formule (IX), on peut citer les monomères :

a) appartenant à la famille des 2-cyanoacrylate de polyfluoroalkyle tels que l'ester 2,2,3,3-tétrafluoropropylique de l'acide 2-cyano-2-propénoïque de formule :

(X)

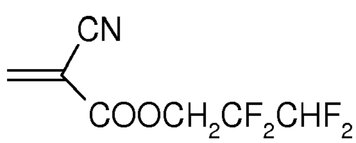

ou encore l'ester 2,2,2-trifluoroéthylique de l'acide 2-cyano-2-propénoïque de formule :

(XI)

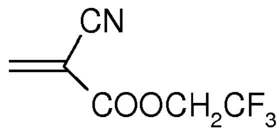

b) les 2-cyanoacrylate d'alkyle ou d'alcoxyalkyle

(XII)

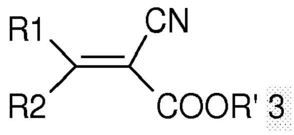

dans laquelle R'3 représente un radical alkyle en C1-C10 ou alcoxy(C1-C4) alkyle(C1-C10), alcényle en C2-C10.

On peut citer plus particulièrement le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de 2-octyle, le 2-cyanoacrylate de 2-propoxyéthyle, le 2-cyanoacrylate de n-octyle le 2-cyanoacrylate d'allyle, le 2-cyanoacrylate de méthoxypropyle et le cyanoacrylate d'iso-amyle, le 2-cyanoacrylate d'allyle.

Dans le cadre de l'invention, on préfère utiliser les monomères b). Selon un mode de réalisation préféré, le ou les monomères cyanoacrylates sont choisis parmi les cyanoacrylates d'alkyle en C6-C10.

Les monomères particulièrement préférés sont les cyanoacrylate d'octyle de formule XIII et leurs mélanges :

(XIII)

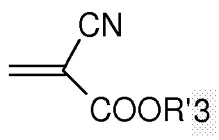

dans laquelle :

R'3 =$-(CH_2)_7-CH_3$,

$-CH(CH_3)-(CH_2)_5-CH_3$,

$-CH_2-CH(C_2H_5)-(CH_2)_3-CH_3$,

$-(CH_2)_5-CH(CH_3)-CH_3$,

$-(CH_2)_4-CH(C_2H_5)-CH_3$.

Les monomères utilisés conformément à l'invention peuvent être fixés de façon covalente sur des supports tels que des polymères, des oligomères ou des dendrimères. Le polymère ou l'oligomère peut être linéaire, ramifié, en peigne ou bloc. La répartition des monomères de l'invention sur la structure polymérique, oligomérique ou dendritique peut être statistique, en position terminale ou sous forme de blocs.

Le ou les monomères cyanoacrylates peut ou peuvent etre présents dans une quantité allant de 0.1 à 80 % en poids, de préference dans une quantité allant de 0.2 à 60 % en poids, et encore plus préférentiellement dans une quantité allant de 0.5 en 50 % en poids, par rapport au poids total de la composition cosmétique.

Dans le cadre de l'invention, les monomères électrophiles sont notamment des monomères capables de polymériser par voie anionique en présence d'un agent nucléophile. Par polymérisation anionique, on entend le mécanisme défini dans l'ouvrage "Advanced Organic Chemistry", Third Edition de Jerry March, pages 151 à 161.

Les agents nucléophiles susceptibles d'initier la polymérisation anionique sont des systèmes connus en eux-mêmes, capables de générer un carbanion au contact d'un agent nucléophile, tels que les ions hydroxyles contenus dans l'eau. On entend par "carbanion ", les espèces chimiques définies dans " Advanced Organic Chemistry, Third Edition ", de Jerry March, page 141.

L'agent nucléophile est un composé moléculaire, un oligomère, un dendrimère ou un polymère possédant des fonctions nucléophiles. De façon non limitative, on peut citer comme fonctions nucléophiles les fonctions : $R_2N^-$, $NH_2^-$, $Ph_3C^-$, $R_3C^-$, $PHNH^-$, pyridine, $ArS^-$, $R-C{\equiv}C^-$, $RS^-$, $SH^-$, $RO^-$, $R_2NH$, $ArO^-$, $N_3^-$, $OH^-$, $ArNH_2$, $NH_3$, $I^-$, $Br^-$, $Cl^-$, $RCOO^-$, $SCN^-$, ROH, RSH, $NCO^-$, $CN^-$, $NO_3^-$, $ClO_4^-$, $H_2O$, Ph représentant un groupe phényle ; Ar représentant un groupe aryle et R représentant un groupe alkyle en C1-C10.

Les monomères électrophiles peuvent être synthétisés selon les méthodes connues décrites dans la technique. En particulier, les monomères cyanoacrylates peuvent être synthétisés selon l'enseignement du US 3 527 224, US 3 591 767, US 3 667 472, US 3 995 641, US 4 035 334 et US 4 650 826.

Selon la présente invention, les monomères sont de préférence choisis parmi les monomères capables de polymériser sur les fibres kératiniques dans des conditions cosmétiquement acceptables. En particulier, la polymérisation du monomère s'effectue de préférence à une température inférieure ou égale à 80°C ce qui n'empêche pas de terminer l'application par un séchage au casque, un brushing ou passage au fer plat ou à friser.

La composition de l'invention contient un agent alcalin azoté du type ammoniaque ou amine ainsi que les compositions tampons comprenant ce type d'agent. L'agent alcalin azoté peut être sous leur forme protonée, étant entendu que le pH de la composition conditionne l'équilibre entre la base et la forme protonée. Dans le cadre de l'invention, on enetnd par agent alcalin azoté ces deux formes de composés, par exemple ammoniaque et ammonium.

L'agent alcalin azoté peut être choisi parmi l'ammoniaque, les amines tels que les amines de petites masses moléculaires telles que les alkylamines primaires, secondaires ou tertiaires, les alcanolamines primaires, secondaires ou tertiaires par exemple l'éthanolamine, les alkyl- ou hydroxyalkyl- di ou tri amines telles que le diaminopropane, de diaminopropanol, les acides aminés, et en particulier les acides aminés basiques tels que lysine et arginine ainsi que les bases azotées du type guanidine. L'agent alcalin azoté peut se trouver sous forme de tampon, en particulier des tampons ammoniacaux. On entend par tampon, un mélange d'agents alcalins azotés tels que définis précédemment avec un acide, et en particulier un acide faible. A titre d'exemple, on peut citer les tampons à base de carbonate ou bicarbonate d'ammonium, de citrate d'ammonium.

Selon un mode de réalisation particulier,l'agent alcalin azoté est l'ammoniaque ou l'éthanolamine.

Selon une variante, l'alcalin azoté est obtenu par un donneur d'alcalin azoté par réaction chimique soit spontanément soit en présence d'un catalyseur ou d'un système enzymatique.

Une seconde variante comprend l'utilisation d'un mélange d'agents alcalins azotés. A titre d'exemple, on peut citer les mélanges ammoniaque/alcanolamine ou ammoniaque/acides aminés.

Le ou les agents alcalins azotés peuvent être combinés à des agents alcalins non azotés tels que soude, potasse...

L'émulsion de la présente invention est en général alcalin, généralement compris entre 8 et 13. Le pH de cette émulsion sera déterminé en fonction de l'utilisation finale de cette émulsion. En particulier, si cette émulsion est destinée à la coloration éclaircissante, le pH de l'émulsion sera compris entre 8 et 11, pour une utilisation finale en défrisage, le pH sera autour de 13, pour un défrisage ou une permanente, il sera compris entre 6 et 10.

L'agent alcalin est préférentiellement introduit dans la phase aqueuse.

La phase aqueuse constitue préférentiellement au moins 50% en poids du poids total de l'émulsion.

La composition de l'invention peut contenir un ou plusieurs inhibiteurs de polymérisation, et plus particulièrement des inhibiteurs de polymérisation anioniques et/ou radicalaires, ceci afin d'accroître la stabilité de la composition dans le temps. De façon non limitative, on peut citer les inhibiteurs de polymérisation suivants : le dioxyde de soufre, l'oxyde nitrique, le trifluorure de bore, l'hydroquinone et ses dérivés tels que l'hydroquinone monéthyléther, la TBHQ, la benzoquinone et ses dérivés tels que la duroquinone, le catéchol et ses dérivés tels que le t-butyl catéchol et le méthoxycatéchol, l'anisole et ses dérivés tels que le méthoxyanisole ou l'hydroxyanisole, le pyrogallol et ses dérivés, le p-méthoxyphénol, l'hydroxybutyl toluène, les alkyl sulfates, les alkyl sulfites, les alkyl sulfones, les alkyl sulfoxydes, les alkyl sulfures, les mercaptans, le 3-sulfonène et leurs mélanges. Les groupements alkyle désignent de préférence des groupement ayant 1 à 6 atomes de carbone.

On peut aussi utiliser à titre d'inhibiteur les acides minéraux ou organiques.

Ainsi la composition cosmétique selon l'invention peut également comprendre au moins un acide minéral ou organique, ce dernier ayant un ou plusieurs groupements carboxyliques ou sulfoniques, présentant un pKa compris entre 0 et 6 tels que l'acide phosphorique, l'acide chlorhydrique, l'acide nitrique, l'acide benzène- ou toluène-sulfonique, l'acide sulfurique, l'acide carbonique, l'acide fluorhydrique, l'acide acétique, l'acide formique, l'acide propionique, l'acide benzoïque, les acides mono-, di- ou trichloroacétiques, l'acide salicylique et l'acide trifluoroacétique, l'acide octanoïque,

l'acide heptanoïque et l'acide hexanoïque.

De préférence, l'acide acétique est utilisé.

La concentration en inhibiteur dans la composition cosmétique de l'invention peut être comprise entre 10 ppm et 30% en poids et plus préférentiellement entre 10 ppm et 15% en poids par rapport au poids total de la composition.

Pour moduler la cinétique de polymérisation anionique, on peut également augmenter la nucléophilie de la fibre par transformation chimique de la matière kératinique.

A titre d'exemple, on peut citer la réduction des ponts di-sulfure composant en partie la kératine en thiols avant application de la composition de l'invention. De façon non exhaustive, on peut citer comme réducteurs des ponts di-sulfure composant en partie la kératine, les composés suivants:

- thiosulfate de sodium anhydre,
- métabisulfite de sodium en poudre,
- thiourée,
- sulfite d'ammonium,
- acide thioglycolique,
- acide thiolactique,
- thiolactate d'ammonium,
- mono-thioglycolate de glycérol,
- thioglycolate d'ammonium,
- thioglycérol,
- acide 2,5-dihydroxybenzoique,
- di-thioglycolate de diammonium,
- thioglycolate de strontium,
- thioglycolate de calcium,
- formo-sulfoxylate de zinc,
- thioglycolate d'isooctyle,
- dl-cystéine,
- thioglycolate de monoéthanolamine.

L'émulsion eau dans l'huile de l'invention peuvent également contenir au moins un agent utilisé habituellement en cosmétique, choisi, par exemple, parmi les agents réducteurs, des corps gras, des plastifiants, des adoucissants, des agents anti-mousse, des agents hydratants, des pigments, des argiles, des charges minérales, des filtres UV, des colloïdes minéraux, des peptisants, des solubilisants, des parfums, des conservateurs, des polymères fixants ou non, des polyols, des protéines, des vitamines, des colorants directs ou d'oxydation, des agents nacrants, des propulseurs, et des épaississants minéraux ou organiques, les cires oxyéthylénées ou non, les paraffines, les acides gras en C10-C30 tels que l'acide stéarique, l'acide laurique, les amides gras en C10-C30 tel que le diéthanolamide laurique, les esters d'alcool gras en C10-C30 tels que les benzoates d'alcool gras en C10-C30 et leurs mélanges.

Selon une variante de l'invention, l'émulsion contient au moins un additif cosmétique choisi parmi les pigments, les nano/micro-objets, les cristaux liquides, les agents oxydants, les colorants directs, les colorants fluorescents, les colorants d'oxydation, les polymères, les agents réducteurs.

Les colorants directs sont par exemple des colorants nitrés de la série benzénique, les colorants directs azoïques, les colorants directs méthiniques, les colorants hydrazones, cationiques ou non cationiques.

Les colorants d'oxydation sont des bases d'oxydation et des coupleurs bien connus de la technique. A titre de coupleurs, on peut notamment citer les méta-phénylènediamines, les méta-aminophénols, les méta-diphénols, les coupleurs naphtaléniques, les coupleurs hétérocycliques et leur sels d'addition. A titre de base d'oxydation, on peut notamment citer les paraphénylènediamines, les bis-phénylalkylènediamines, les paraaminophénols, les ortho-aminophénols et les bases hétérocycliques.

Les pigments utiles sont connus de la technique, ils sont notamment décrits dans l'encyclopédie de technologie chimique de Kirk-Othmer et dans l'encyclopédie de chimie industrielle de Ullmann.

Ces pigments peuvent se présenter sous forme de poudre ou de pâte pigmentaire. Les pigments peuvent par exemple être choisis parmi les pigments blancs ou colorés, les laques, les pigments à effets spéciaux tels que les nacres ou les paillettes, et leurs mélanges.

Par polymère on entend tous les polymères utilisables en cosmétique, naturels ou synthétiques et en particulier les polymères obtenus par polymérisation radicalaire ou anionique ou par polycondensation ou par ouverture de cycles. Ces polymères peuvent être linaires, ramifiés ou en étoile.

On utilisera de préférence les polymères naturels modifiés ou non chimiquement comme les dextrans, les celluloses (les carboxymethylhdroxypropylcellulose), les guars (les carboxymethylhdroxypropylguars) les amidons, les alginates, les chitosanes...

Les pigments et les charges peuvent être enrobées par des composés organiques ou minéraux.

Préférentiellement les pigments et les charges sont enrobés par des corps gras comme par exemple du parleame, des composé siliconés, des acides gras, des alcools gras comme l'acide et l'alcool palmitique, l'acide et l'alcool stéarique, et leur mélange. Les acides gras pouvant être sous forme de sels de sodium, de potassium, de magnésium, de fer, de titane, de zinc ou d'aluminium.

L'émulsion eau dans l'huile de l'invention peut être formulée sous différentes formes galéniques tels qu'une lotion, une mousse aérosol, un après shampooing ou un shampooing, un gel, une cire.

L'émulsion eau dans l'huile de l'invention est une émulsion cosmétique. On entend par cosmétique dans le cadre de l'invention une émulsion compatible avec les matières kératiniques.

On peut préparer plusieurs émulsions du type eau dans huile (E /H) de composition différentes et les mélanger dans des proportions variables pour obtenir une nouvelle émulsion contenant différents actifs cosmétiques.

Le procédé de la présente invention comprend l'application de l'émulsion eau dans l'huile décrite précédemment sur les fibres kératiniques.

L'émulsion de la présente invention peut par exemple être utilisée pour la décoloration des fibres kératiniques. Dans ce cas, l'émulsion elle-même peut contenir un agent oxydant ou l'agent oxydant peut être ajouté extemporanément au moment de l'emploi. Les agents oxydants classiquement utilisés sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases.

L'émulsion de la présente invention peut être utilisée pour la coloration d'oxydation. Dans ce cas, l'émulsion peut contenir un agent oxydant ou un précurseurs de colorant d'oxydation. On peut aussi ajouter à l'émulsion de la présente invention au moment de l'emploi une composition comprenant le colorant d'oxydation et/ou une composition comprenant l'agent oxydant.

L'émulsion de la présente invention peut être utilisée pour la coloration directe. Dans ce cas, le colorant direct peut être présent dans l'émulsion de l'invention ou être ajouté à l'émulsion au moment de l'emploi. Dans le cas de la coloration éclaircissante directe, l'émulsion de la présente invention peut contenir le colorant direct et l'agent oxydant. Selon une variante, le colorant direct et/u l'agent oxydant sont ajoutés au moment de l'emploi à l'émulsion de la présente invention.

L'émulsion de la présente invention peut être utilisée pur la permanente, le défrisage thiolé. Dans ce cas, l'émulsion peut comprendre un ou plusieurs réducteurs tels que l'acide thioglycolique, la cystéine. Le réducteur peut être ajouté à l'émulsion au moment de l'emploi.

L'émulsion de l'invention peut être utilisée dans le cadre du défrisage alcalin. Dans ce cas, l'émulsion peut contenir une ou plusieurs bases minérales telles que la soude, la lithine, le citrate d'ammonium, les sels de guanidinium. Ces bases minérales peuvent aussi être ajoutées à l'émulsion au moment de l'emploi.

L'émulsion de l'invention peut être utilisée dans des compositions de coiffage, de soin ou d'hygiène. On y ajoute soit directement, soit sous forme de mélange au moment de l'emploi, des épaississants tels que les carbopols, les polymères tels que les polymères anioniques comme les polyacryliques ou méthacryliques, des tensio actifs anioniques, des tensio actifs cationiques, des tensio actifs amphotère ou non ionique.

Selon le procédé de l'invention, l'émulsion ou la composition prête à l'emploi résultant du mélange de l'émulsion avec une composition cosmétique telle que décrite précédemment peut être appliqué sur les cheveux secs ou préalablement humidifiés ou shampooinés. Les cheveux peuvent être alors séchés (sèche cheveux, casque ou fer). Un shampooing terminal peut être éventuellement effectué.

Selon une variante, les cheveux sont pré-traités par au moins un agent nucléophile. Les cheveux peuvent être séchés. L'émulsion de l'invention peut être alors appliquée le cheveu. Les cheveux sont séchés (sèche cheveux, casque ou fer). Un shampooing terminal peut être éventuellement effectué.

Selon une autre variante, les cheveux sont pré-traités par au moins un additif cosmétique tel que défini précédemment. Les cheveux peuvent être séchés, puis l'émulsion de l'invention est appliquée sur le cheveu. Les cheveux sont séchés (sèche cheveux, casque ou fer). Un shampooing terminal peut être éventuellement effectué.

Selon un mode de réalisation, le procédé de l'invention consiste à combiner l'application de l'émulsion de l'invention avec des traitements de la fibre tels que décrits précédemment.

Ainsi selon cette variante, on peut appliquer en pré traitement un réducteur de permanente ou une permanente ou une coloration d'oxydation ou une décoloration ou un shampooing ou un produit de coiffage ou un défrisage alcalin en pré traitement étant suivi par l'application de l'émulsion de l'invention.

Selon une autre variante, l'émulsion de l'invention est appliquée en pré-traitement afin de protéger la fibre kératinique avant l'application de traitements connus pour être susceptible de dégrader les cheveux. On peut ensuite éventuellement démaquiller la fibre.

Ainsi après avoir appliqué la composition de l'invention selon un des procédés déjà décrits on applique un réducteur de permanente ou une permanente ou une coloration d'oxydation ou une décoloration ou un défrisage alcalin.

L'application de l'émulsion de l'invention peut aussi être appliquée après une coloration d'oxydation ou directe ou un mélange de ces colorants afin de protéger l'éclat de la couleur aux shampooings.

La présente invention a aussi pour objet un kit comprenant une première émulsion telle que décrite précédemment et une seconde composition comprenant l'agent nucléophile.

L'invention a aussi pour objet un kit comprenant une première composition contenant au moins un monomère cyanoacrylate et éventuellement au moins un inhibiteur de polymérisation anionique et/ou radicalaire et/ou un acide et une deuxième composition contenant une émulsion eau dans huile et éventuellement un acide organique.

Selon un autre mode de réalisation, une première composition comprend une émulsion eau dans l'huile qui comprend une phase aqueuse une phase huileuse, un tensio-actif et un monomère cyanoacrylate, et éventuellement un acide et une seconde composition comprend l'agent alcalin azoté.

Selon une variante, la première composition contient le monomère cyanoacrylate et éventuellement un acide et la seconde composition comprend une phase aqueuse une phase huileuse, un tensio-actif et l'agent alcalin azoté sous forme de l'émulsion eau dans l'huile de l'invention (phase aqueuse, phase huileuse, tensio-actif et agent alcalin azoté).

Selon un autre variante, une première composition comprend un monomère de cyanoacrylet et éventuellement un acide, une deuxième composition comprend un agent alcalin azoté et une troisième composition sous forme d'émulsion eau dans l'huile comprenant une phase aqueuse, une phase huileuse et un tensio-actif.

Le procédé de l'invention peut être mis en oeuvre plusieurs fois afin de réaliser des applications multiples de l'émulsion de l'invention afin d'obtenir une superposition de couches pour atteindre des propriétés spécifiques du gainage en terme de nature chimique, résistance mécanique, épaisseur, aspect, toucher.

**EXEMPLE 1**

Le tableau suivant illustre des exemples d'émulsions selon la présente invention dans lesquelles de l'ammoniaque à 2 % est utilisé en tant qu'agent alcalin azoté est introduit dans la phase aqueuse. Pour chacune de ces émulsions, l'odeur d'ammoniaque est quasiment supprimée.

| **Emulsion H/E siliconée** | Ex. 1.1 | Ex. 1.2 | Ex.1.3 | Ex. 1.4 | Ex.1.5 | Ex. 1.6 | Ex.1.7 | Ex. 1.8 |
|---|---|---|---|---|---|---|---|---|
| 1-méthyl heptyl cyanoacrylate | 9,10 % | 4,80 % | 9,10 % | 4,80 % | 4,20 % | 10 % | 5 % | 10 % |
| Cyclopenta siloxane | 13,15 % | 6,90 % | 13,15 % | 6,90 % | 7 % | 10 % | 7 % | 5 % |
| PEG/PG-18/18 diméthicone | 0,45% | 0,20 % | 0,45 % | 0,24 % | 0,50 % | 1 % | 0,50 % | 0,50 % |
| Acide acétique | 27,30 % | 14,30 % | 9,10 % | 4,76 % | 2,70 % | 1,50 % | 1,50 % | 1,50 % |
| Eau | 50 % | 74 % | 68 % | 83 % | 86 % | 78 % | 86 % | 83 % |
| | | | | | | | | |
| *Phase grasse* | 22,70 % | 11,90 % | 22,70 % | 11,90 % | 11,70 % | 21 % | 12,50 % | 15,50 % |
| *Phase aqueuse* | 77,30 % | 88,10 % | 77,30 % | 88,10 % | 88,30 % | 79 % | 87,50 % | 84,50 % |

| **Emulsion E/H non siliconée** | Ex. 1.9 | Ex. 1.10 |
|---|---|---|
| 1-méthyl heptyl cyanoacrylate | 5 % | 5 % |
| Mineral oil / paraffinium liquidium | 7% | 5% |
| Olea europaea (olive) fruit oil / olea europaea | - | 2 % |
| Hydroxyethyldiethonium polyisobutenyl triethylaminosuccinate | 1 % | 1 % |
| Acide acétique | 3% | 3% |
| Eau | 84% | 84% |
| | | |

(suite)

| **Emulsion E/H non siliconée** | Ex. 1.9 | Ex. 1.10 |
|---|---|---|
| *Phase grasse* | 13 % | 13 % |
| *Phase aqueuse* | 87% | 87 % |

**EXEMPLE 2 : Limitation de l'odeur d'ammoniaque dans une formule capillaire éclaircissante**

Une émulsion « E » eau dans huile à 90g% est préparée comme suit :

Phase grasse: dans un bécher A, on introduit 2,8g de cyclopentasiloxane (100% MA) et 8.33g d'un émulsionnant E/H constitué de poly dimethylsiloxane oxyethylene oxypropylene (18 OE/18 OP) + cyclopenta dimethylsiloxane + eau (10/88/2)

Phase aqueuse : dans un bécher B, on introduit 10,2g d'ammoniaque (solution à 20.5g% d'ammoniac) et 68,67g d'eau

Sous agitation au Rayneri (900/1400 tr/min), la phase aqueuse B est progressivement introduite dans la phase grasse A.

On ajoute à l'émulsion inverse « E » 10g% de 2-octylcyanoacrylate. 5 g de la composition obtenue est mélangée avec 5g d'oxydant 40 volumes (12g% d'eau oxygénée).
Le mélange « C1 » ainsi obtenu ne sent pas l'ammoniaque.

Parallèlement, on mélange 5g de l'émulsion inverse « E » à 5g d'oxydant 40 volume (12g% d'eau oxygénée). Le mélange obtenu, « C2 », sent fortement l'ammoniaque.

Le pouvoir éclaircissant du mélange C1 est comparé à celui du mélange C2. Pour cela, chaque mélange est appliqué sur 1 g de mèche de cheveu châtain (hauteur de ton = 4). Le temps de pose est de 30 min à température ambiante. Enfin, la mèche est lavée et subit un shampooing. Le niveau d'éclaircissement des mèches a été évaluée dans le système L* a* b*, au moyen d'un spectrophotomètre CM 2002 MINOLTA ®, (Illuminant D65). Dans ce système L* a* b*, L* représente l'intensité de la couleur, a* indique l'axe de couleur vert/rouge et b* l'axe de couleur bleu/jaune. Plus la valeur de L est faible, plus la couleur est foncée ou très intense. Plus la valeur de a* est élevée plus la nuance est rouge et plus la valeur de b* est élevée plus la nuance est bleue. Les mesure de L*a*b* ont été réalisées sur les mèches avant et après éclaircissement. Le niveau d'éclaircissement est mesuré par le paramètre ΔE selon l'équation suivante :

$$\Delta E = \sqrt{(L_1 - L_0)^2 + (a_1^* - a_0^*)^2 + (b_1^* - b_0^*)^2}$$

$L_0$, $a_0^*$ et $b_0^*$ définissent les valeurs colorimétriques avant éclaircissement et $L_1$, $a_1^*$ et $b_1^*$ défissent les valeurs colorimétriques après éclaircissement.

Comme l'indique le tableau ci-dessous, la mèche traitée avec le mélange C1 a un niveau d'éclaircissement comparable à celui de la mèche traitée avec le mélange C2.

| | L* | a* | b* | **ΔE*ab** |
|---|---|---|---|---|
| Cheveu naturel HT4 | 21.61 | 2.68 | 3.06 | |
| Cheveu naturel HT4 traité avec le mélange C1 | 25.66 | 6.25 | 8.52 | **7.68** |
| Cheveu naturel HT4 traité avec le mélange C2 | 26.51 | 6.34 | 8.21 | **8.00** |

**Revendications**

1. Emulsion eau dans l'huile pour le traitement des fibres kératiniques comprenant au moins un tensio-actif ; une phase aqueuse comprenant au moins un agent alcalin azoté et une phase huileuse comprenant un ou plusieurs monomères cyanoacrylates capables de polymériser par voie anionique en présence d'un agent nucléophile.

2. Emulsion eau dans l'huile dans laquelle la phase huileuse comprend un ou plusieurs solvants organiques liquides.

3. Emulsion eau dans l'huile selon la revendication 1 ou 2 dans laquelle le solvant organique est choisi parmi .les alcools aromatiques ; les alcools gras liquides , les polyols ; les silicones volatiles, les huiles minérales, organiques ou végétales, les alcanes ; les acides gras liquides, les esters gras liquides.

4. Emulsion eau dans l'huile selon l'une quelconque des revendications 1 à 3 dans laquelle le solvant organique comprend une silicone volatile, seule ou en mélange avec d'autres silicones.

5. Emulsion eau dans l'huile selon l'une quelconque des revendications précédentes dans laquelle le monomère cyanoacrylate est choisi parmi les monomères de formule (I) :

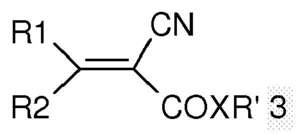

(I)

dans laquelle :

X désigne NH, S ou O,
R1 et R2 désignent chacun, indépendamment l'un de l'autre, un groupe peu ou non électro-attracteur (peu ou non inductif-attracteur) tel que :

- un atome d'hydrogène,
- un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR, -COOR, -COR, -SH, - SR, -OH, et les atomes d'halogène,
- un résidu polyorganosiloxane modifié ou non,
- un groupement polyoxyalkylène,

R désigne un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR', - COOR', -COR', -SH, -SR', -OH, les atomes d'halogène, ou un résidu de polymère pouvant être obtenu par polymérisation radicalaire, par polycondensation ou par ouverture de cycle, R' désignant un groupe alkyle en C1-C10,
R'3 représentant un atome d'hydrogène ou un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR', -COOR', -COR', -SH, -SR', -OH, les atomes d'halogène, ou un résidu de polymère pouvant être obtenu par polymérisation radicalaire, par polycondensation ou par ouverture de cycle, R' désignant un groupe alkyle en C1-C10.

6. Emulsion eau dans l'huile selon la revendication 5 dans laquelle le monomère cyanoacrylate est choisi parmi les monomères de formule (IV) :

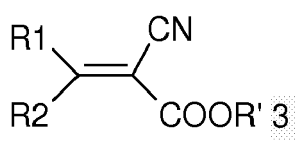

(IV)

dans laquelle R’3 représente un radical alkyle en C1-C10, alcényle en C2-C10, ou alcoxy(C1-C4) alkyle(C1-C10).

7. Emulsion eau dans l’huile selon la revendication 6 dans laquelle le monomère cyanoacrylate est choisi parmi le 2-cyanoacrylate d’éthyle, le 2-cyanoacrylate de méthyle, le 2-cyanoacrylate de n-propyle, le 2-cyanoacrylate d’isopropyle, le 2-cyanoacrylate de tert-butyle, le 2-cyanoacrylate de n-butyle, le 2-cyanoacrylate d’iso-butyle, le cyanoacrylate de 3-méthoxybutyle, le cyanoacrylate de n-décyle, le 2-cyanoacrylate d’hexyle, le 2-cyanoacrylate de 2-éthoxyéthyle, le 2-cyanoacrylate de 2-méthoxyéthyle, le 2-cyanoacrylate de 2-octyle, le 2-cyanoacrylate de 2-propoxyéthyle, le 2-cyanoacrylate de n-octyle, le cyanoacrylate d’iso-amyle, le 2-cyanoacrylate d’allyle et le 2-cyanoacryale de methoxypropyle.

8. Emulsion eau dans l’huile selon la revendication 6 dans laquelle le monomère cyanoacrylate est choisi parmi les cyanoacrylates d’alkyle en C6-C10.

9. Emulsion eau dans l’huile selon la revendication 6 dans laquelle le monomère cyanoacrylate est choisi parmi les monomères les cyanoacrylates d’octyle de formule (V) et leurs mélanges :

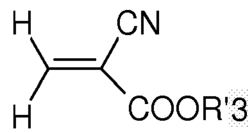

(V)

dans laquelle : R’3 =-(CH2)7-CH3,

- CH(CH3)-(CH2)5-CH3,
- CH2-CH(C2H5)-(CH2)3-CH3,
- (CH2)5-CH(CH3)-CH3,
- (CH2)4-CH(C2H5)-CH3.

10. Emulsion eau dans l’huile selon l’une quelconque des revendications précédentes, **caractérisée en ce que** la teneur en monomère cyanoacrylate varie en une quantité allant de 0,1 à 80 % en poids, de préférence en une quantité allant de 0,2 à 60 % en poids, et encore plus préférentiellement en une quantité allant de 0,5 en 50 % en poids, par rapport au poids total d’émulsion.

11. Emulsion eau dans l’huile selon l’une quelconque des revendications précédentes dans laquelle l’agent alcalin azoté est choisi parmi l’ammoniaque, les alkylamines, les alcanolamines, les alkyl- ou hydroxyalkyl- di ou tri amines, les bases azotées.

12. Emulsion eau dans l’huile selon l’une quelconque des revendications précédentes dans laquelle l’agent alcalin azoté est l’ammoniaque ou l’éthanolamine.

13. Emulsion eau dans l’huile selon l’une quelconque des revendications précédentes dans laquelle l’alcalin azoté est obtenu par un donneur d’alcalin azoté par réaction chimique soit spontanément soit en présence d’un catalyseur ou d’un système enzymatique.

14. Emulsion eau dans l’huile selon l’une quelconque des revendications précédentes comprenant de plus un inhibiteur de polymérisation choisi parmi le dioxyde de soufre, l’oxyde nitrique, le trifluorure de bore, l’hydroquinone et ses dérivés, la benzoquinone et ses dérivés, le catéchol et ses dérivés, l’anisole et ses dérivés , le pyrogallol et ses dérivés, le p-méthoxyphénol, l’hydroxybutyl toluène, les alkyl sulfates, les alkyl sulfites, les alkyl sulfones, les alkyl sulfoxydes, les alkyl sulfures, les mercaptans, le 3-sulfonène, les acides organiques et leurs mélanges.

15. Emulsion eau dans l’huile selon la revendication 14 dans laquelle l’inhibiteur de polymérisation est l’acide acétique.

16. Emulsion eau dans l’huile selon l’une quelconque des revendications précédentes dans laquelle le ou les tensio-actifs présents dans l’émulsion sont choisis parmi les tensio-actifs siliconés.

**17.** Emulsion eau dans l'huile selon l'une quelconque des revendications 1 à 16 dans laquelle le tensio actif est non siliconé.

**18.** Emulsion eau dans l'huile selon la revendication 16 ou 17 dans laquelle les tensio-actifs sont présents dans l'émulsion de la présente invention dans des proportions variant de 0,1% à 30% en poids d'émulsion.

**19.** Emulsion eau dans l'huile selon l'une quelconque des revendications précédentes dans laquelle la phase aqueuse représente au moins 50 % en poids du poids total de l'émulsion.

**20.** Emulsion eau dans l'huile selon l'une quelconque des revendications précédentes dans laquelle le tensio-actif est non ionique et présente une HLB inférieure à 10.

**21.** Procédé de traitement des matières kératiniques **caractérisé par le fait que** l'on applique sur les fibres kératiniques l'émulsion telle que définie à l'une quelconque des revendications 1 à 20.

**22.** Procédé selon la revendication 21 comprenant un pré-traitement avec un agent nucléophile..

**23.** Kit de traitement des fibres kératiniques comprenant une première composition qui contient l'émulsion telle que définie à l'une quelconque des revendications précédentes 1 à 20 et une seconde composition qui contient un agent nucléophile.

**24.** Kit de traitement des fibres kératiniques comprenant une première composition contenant au moins un monomère cyanoacrylate tel que défini à l'une quelconque des revendications 1 à 10 et éventuellement au moins un inhibiteur de polymérisation anionique et/ou radicalaire et/ou un acide et une deuxième composition contenant une émulsion eau dans huile comprenant une phase aqueuse comprenant un agent alcalin azoté et une phase huileuse, un tensio-actif et éventuellement un acide organique.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 2528378 A
- US 2781354 A
- US 4874554 A
- US 4137180 A
- FR 2818902
- US 3527224 A
- US 3591767 A
- US 3667472 A
- US 3995641 A
- US 4035334 A
- US 4650826 A


### Littérature non-brevet citée dans la description


- **TODD ; BYERS.** Volatile Silicone fluids for cosmetics. *Cosmetics and toiletries,* Janvier 1976, vol. 91, 27-32
- **M.R. PORTER.** Handbook of Surfactants. 1991, 116-178
- **PR WELLS.** *Prog. Phys. Org. Chem.,* 1968, vol. 6, 111
- **JERRY MARCH.** Advanced Organic Chemistry. 151-161
- **JERRY MARCH.** Advanced Organic Chemistry. 141